# EUROPEAN PATENT SPECIFICATION

(11) **EP 0 441 644 B1**
(45) Date of publication and mention of the grant of the patent: **11.09.1996**
(21) Application number: 91301036.9
(22) Date of filing: 08.02.1991
(51) Int. Cl.: C12Q 1/70, G01N 33/576, C07K 14/00, C07K 7/08, C12N 15/40, C12P 21/08, C12N 15/51, C12N 15/62

(54) **Non-A, non-B hepatitis related nucleic acids, antigens, antibodies and their detection reagents**
Nukleinsäuren, Antigene und Antikörper betreffend Non-A, Non-B Hepatitis und deren Nachweisreagenzien
Acides nucléiques, antigènes et anticorps associés avec l'hépatite non-A, non-B et leurs réactifs de détection

(30) Priority: 09.02.1990 JP 28191/90; 14.06.1990 JP 153887/90
(43) Date of publication of application: 14.08.1991
(73) Proprietor: IMMUNO JAPAN INC., Tokyo 167 (JP)
(72) Inventor: Nakamura, Tetsuo, Suginami-ku Tokyo 167 (JP); Mishiro, Shunji, Bunkyo-ku Tokyo (JP)
(74) Representative: Arthur, Bryan Edward

(56) References cited:
- EP-A- 0 318 216
- THE LANCET, vol. 336, no. 8728, 8 December 1990, LONDON, GB pages 1400 - 1403; S. MISHIRO ET AL.: 'Non-A, non-B hepatitis specific antibodies directed at host-derived epitope: implication for an autoimmune process'

## Description

### Introduction to the Invention

The present invention relates to novel nucleic acids, antigens, antibodies and non-A, non-B hepatitis diagnostic reagents using those materials.

The causative agent of non-A, non-B hepatitis afflicts human beings with such diseases as acute hepatitis, fulminant hepatitis, chronic hepatitis, liver cirrhosis and hepatocellular carcinoma. It presents itself as the major cause of post transfusion hepatitis in approximately 10% of the blood recipients in Japan. The causative viruses of hepatitis A and hepatitis B have been isolated and these diseases are under medical control. However, the causative agent of non-A, non-B hepatitis is a mystery although its presence was assumed over 10 years ago.

In 1988, a research group at Chiron Corporation in the United States announced that they had succeeded in cloning the gene of the causative agent, and introduced an inmunoassay kit for detection of viral antibody specific to non-A, non-B hepatitis. That diagnostic reagent is now being evaluated at various hospitals, research institutions and blood centers. According to Chiron's research group, the causative agent is a Flavivirus because of its genomic structure, and they have named it hepatitis C virus (HCV). The data reported to date have confirmed a good correlation between HCV antibody detected by their immunoassay kit and the course of the disease of non-A, non-B hepatitis. With respect to the HCV antibody, however, there are problems yet to be solved and elucidated: its insufficient specificity and sensitivity (revealed in several reports), cross reaction between the HCV and a large part of auto-immune hepatitis and hepatitis B, and proof that what they call HCV is the real causative agent of non-A, non-8 hepatitis.

The inventors have been strenuously conducting research for isolation of this causative agent, and, after more than 10 years of untiring efforts, have succeeded in completing this invention. The non-A, non-B specific diagnostic assay of the present invention can detect non-A, non-8 hepatitis in patients which the kit using Chiron's antigen (Ortho HCV Ab ELISA test, Ortho Diagnostic Systems, Tokyo, Japan) fails to detect.

### SUMMARY OF THE INVENTION

An object of the present invention is to provide previously unidentified nucleic acids, antigens and antibodies required for detection of non-A, non-B hepatitis.

Base sequences and amino acid sequences of nucleic acids and proteins disclosed in the present invention are in no way homologous to those of Chiron's HCV genome (European Patent Application No. 88310922.5/EP-A-0318216).

Nucleic acids and proteins disclosed in the present invention have usefulness in the diagnosis of non-A, non-B hepatitis and have been provided only by this invention as totally new materials.

The present invention discloses ribonucleic acids GOR47-1 RNA and GOR47-1 RNAC and deoxyribonucleic acids GOR47-1 DNA and GOR47-1 DNAC; polypeptide GOR47-1 PROTEIN, oligopeptide spGOR1, oligopeptide spGOR2 and oligopeptide spGOR-epi; detection reagents for non-A, non-B hepatitis related genes using each of the aforementioned nucleic acids as probe or primer; detection reagents for antibody associated with non-A, non-B hepatitis using the aforementioned proteins or fusion protein of such proteins and conventional proteins; monoclonal and polyclonal antibodies specific to non-A, non-B hepatitis obtained by immunization of animals with the aforementioned peptide(s) as antigen; and detection reagents of non-A, non-B hepatitis related antigens using the aforementioned specific antibodies.

Nucleic acids and peptides under this invention are all new materials.

### BRIEF DESCRIPTION OF THE DRAWINGS

Fig. 1 shows the base sequence of recombinant lambda-gt11 phage DNA to assemble GOR-47 DNA in EcoRI cleavage. Sequence within the frame is GOR47-1 DNA and those on the left and right sides of the frame are lambda-gt11 DNA.

Fig. 2 shows ORF and amino acid sequence of fusion protein produced by recombinant GOR47-1·lambda-gt11. Sequence within the frame is ORF and amino acid sequence of GOR47-1 PROTEIN.

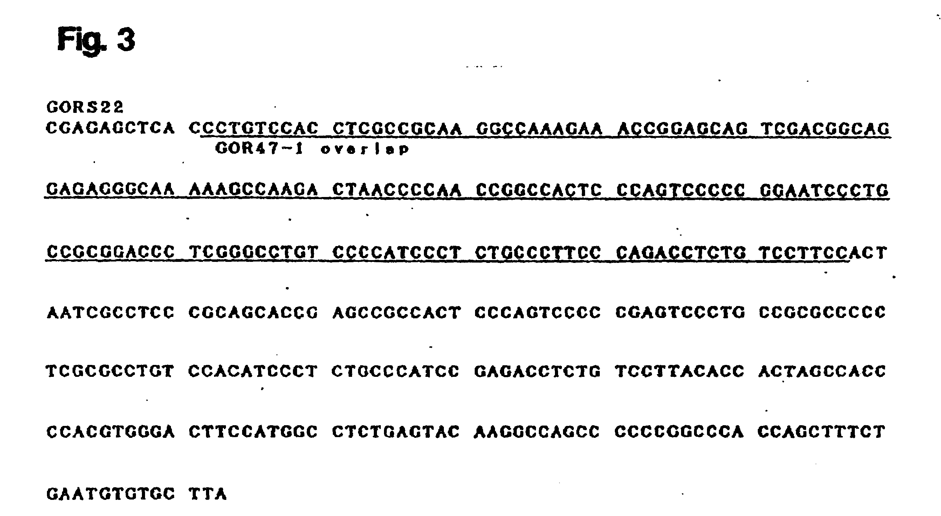

Fig. 3 is the nucleotide sequence of GORS22, sister clone of GOR47-1. Sequence underlined overlaps with that of GOR47-1.

Fig. 4 shows GOR47-1 related ORF encoded by GOR22 and its amino acid sequence. (Underlined sequence shows the common sequence with GOR47-1.)

Fig. 5 shows G1 and G2 used as primers for PCR.

Fig. 6 shows agarose gel electrophoresis patterns of the material produced by PCR.

Fig. 7 is distribution pattern of chronic non-A, non-B hepatitis patient sera showing positive for NANB-GOR antibody. Sera positive for Chiron's HCV antibody are shown by * mark.

Fig. 8 shows reactivity the partial peptide on the ORF of GOR47-1 determined by EIA. Samples with ○ are non-A, non-B hepatitis and those with ● are hepatitis B derived; βGal/GOR is fusion protein of β-galactosidase and GOR47-1; spGOR3 is the peptide extended by 26 residues from the 15th residue of spGOR-epi toward C terminus and having the sequence LPVPRNPCRGPSGLSPSLCPSQTSVL.

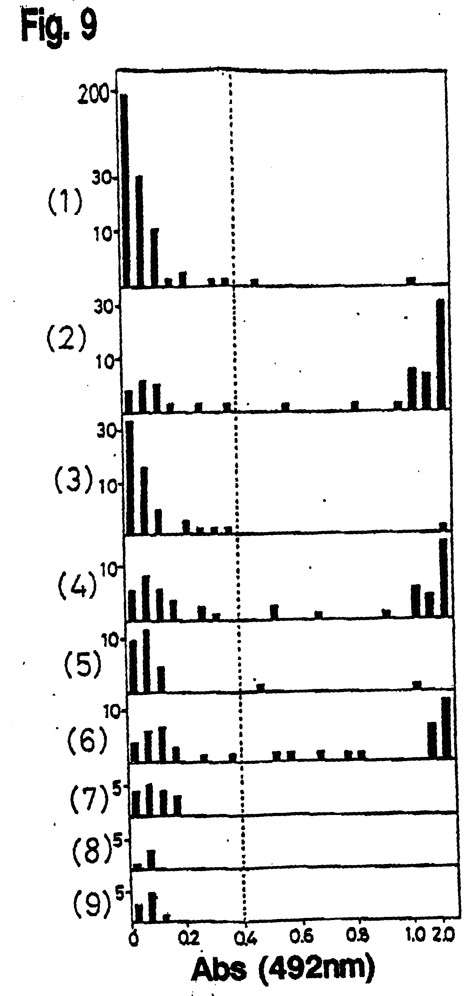

Fig. 9 is a histogram showing NANB-GOR antibody in samples from normal subjects and chronic hepatitis patients detected by EIA using spGOR2 as antigen. On the ordinate is samples from normal subjects and chronic hepatitis patients detected by EIA using spGOR2 as antigen. on the ordinate is shown frequency. Absorbance 0.4 at OD492 is tentatively set as the cut-off value and shown by a dotted line. Positive rate for each group is as follows: (1) Normal subject-1%; (2) non-A, non-B chronic hepatitis patients-76%; (3) Chronic hepatitis B patients-2%; (4) non-A, non-B liver cirrhosis56%; (5) Hepatitis B liver cirrhosis-7%; (6) non-A, non-B hepatoma-56%; (7) Hepatitis B hepatoma-0%; (8) Lupoid hepatitis-0%; (9) Primary biliary cirrhosis-0%.

Fig. 10 shows change with time of NANB-GOR antibody in acute non-A, non-B hepatitis patients determined by EIA using spGOR2. O : IgG class NANB-GOR antibody; □ : IgM class NANB-GOR antibody; ● : HCV by Ortho HCV ELISA Ab test kit; black area: Serum ALT (alanine aminotransferase) value.

### DETAILED DESCRIPTION OF THE INVENTION

Features of the present invention reside in the following:

Ribonucleic Acid GOR47-1 RNA having the following nucleotide mequence:

C C U G U C C A C C U C G C C G C A A G
G C C A A A G A A A C C G G A G C A G U
C G A C G G C A G G A G A G G G C A A A
A A G C C A A G A G U A A C C C C A A C
C G G C C A C U C C C A G U C C C C C G
G A A U C C C U G C C G C G G A C C C U

C G G G C C U G U C C C C A U C C C U C
U G C C C U U C C C A G A C C U C U G U
C C U U C C.

Ribonucleic Acid GOR47-1 RNAC having the following nucleotide sequence:

G G A A G G A C A G A G G U C U G G G A
A G G G C A G A G G G A U G G G G A C A
G G C C C G A G G G U C C G C G G C A G
G G A U U C C G G G G A C U G G G A G
U G G C C G G U U G G G U U A C U C U
U G G C U U U U U G C C C U C U C C U G
C C G U C G A C U G C U C C G G U U U C
U U U G G C C U U G C G G C G A G G U G
G A C A G G.

GOR47-1 RNAC is the complementary strand of GOR47-1 RNA.

Deoxyribonucleic Acid GOR47-1 DNA having the following nucleotide sequence:

C C T G T C C A C C T C G C C G C A A G
G C C A A A G A A A C C G G A G C A G T
C G A C G G C A G G A G A G G G C A A A
A A G C C A A G A G T A A C C C C A A C
C G G C C A C T C C C A G T C C C C C G
G A A T C C C T G C C G C G G A C C C T
C G G G C C T G T C C C C A T C C C T C

T G C C C T T C C G A G A C C T C T G T
C C T T C C.

GOR47-1 DNA is a DNA counterpart of GOR47-1 RNA.

Deoxyribonucleic acid GOR47-1 DNAC having the following nucleotide sequence:

G G A A G G A C A G A G G T C T G G G A
A G G G C A G A G G G A T G G G A C A
G G C C C G A G G G T C C G C G G C A G
G G A T T C C G G G G G A C T G G G A G
T G G C C G G T T G G G G T T A C T C T
T G G C T T T T T G C C C T C T C C T G
C C G T C G A C T G C T C C G G T T T G
T T T G G C C T T G C G G C G A G G T G
G A C A G G.

GOR47-1 DNAC is the complementary strand of GOR47-1 DNA.

Additional features include the following:

Protein GOR47-1 PROTEIN having the following amino acid sequence:

C P P R R K A K E T G A V D G R R G Q K
A K S N P N R P L P V P R N P C R G P S
G L S P S L C P S Q T S V L P.

Oligopeptide spGOR1 having the following amino acid sequence:

C P P R R K A K E T G A V D G R R G Q K
A K S N P N R P L.

Oligopeptide spGOR2 having the following amino acid sequence:

G R R G Q K A K S N P N R P L P V P R N
P C R G P S G.

Oligopeptide spGOR-epi having the following amino acid sequence:

G R R G Q K A K S N P N R P L.

In the above sequences, A, G, C, and U stand for Adenine, Guanine, Cytosine and Uracil respectively in the ribonucleic acids of this invention.

A, G, C, and T stand for Adenine, Guanine, Cystosine and Thymine respectively in the deoxyribonucleic acids of this invention.

C, P, R, K, A, E, T, G, V, D, Q, S, N, L, stand for Cysteine, Proline, Arginine, Lysine, Alanine, Glutamic acid, Threonine, Glycine, Valine, Aspartic acid, Glutamine, Serine, Asparagine and Leucine respectively in the proteins of this invention.

The following procedures were utilized:

### (1) Preparation of animal models for non-A, non-B hepatitis

Research started with preparation of animal models for non-A, non-B hepatitis. Various animals were injected with sera from donors which were known to have caused post transfunional non-A, non-B hepatitis in man. Among those animals, only chimpanzees responded and expressed symptoms similar to human non-A, non-B hepatitis. In case of the experimental non-A, non-B hepatitis in chimpanzees, some characteristic ultrastructural changes tend to appear in the cytoplasm of liver cells before clinical symptoms. Making this ultrastructural change a reliable marker, experimental passage of non-A, non-B causative agent was undertaken from a human being to a chimpanzee, then from the chimpanzee to other chimpanzees.

### (2) Material for isolation of non-A, non-B hepatitis related gene

Human serum (No. 30017) was injected to a chimpanzee (C37) and the serum from that chimpanzee in acute phase was injected to 5 chimpanzees (C41, C43, C45, C46 and C47). Among those 5 chimpanzees, plasma were take from those in the phase positive for the aforementioned ultrastructural changes and pooled for injection to other animals including the chimpanzee CH19. Plasma taken from CH 19 in acute phase of non-A, non-B hepatitis was used as the material for isolation of non-A, non-B hepatitis related gene.

### (3) Extraction of nucleic acids form chimpanzee plasma

About 6 liters of plasma from the chimpanzee (CH19) in acute phase of non-A, non-B hepatitis was centrifuged on the J6 rotor (Beckman) at 3,000 rpm for 30 minutes, and the resulting supernatant was centrifuged on the Ti-15 rotor (Beckman) at 4°C at 30,000 rpm for 5.3 hours. The resulting precipitate was suspended in 120 ml of 50 mM Tris-Cl (pH7.5)/5mM EDTA to obtain virus-rich fraction concentrated by approximately 50 times. After addition to 6 ml of this fraction (equivalent to 300 ml of the original plasma) of 6 ml of SDS/Proteinase K cocktail (400mM NaCl/20mM EDTA/4% SDS/100mM Tris-Cl buffer (pH 8.0)/Proteinase K 2 mg/ml) and overnight incubation at 37°C, nucleic acids were extracted with phenol and precipitated by ethanol.

### (4) cDNA synthesis

Using 1/4 in volume (equivalent to 75 ml of the original plasma) of the nucleic acids obtained under (3) above, cDNA was synthesized. After incubation of the template RNA at 65°C for 3 minutes, primary cDNA strand was synthesized in two tubes, one tube with oligo-dT₁₂, and the other with randam-hexanucleotide as primer. This series of cDNA synthesis, including synthesis of the secondary cDNA strand and blunting of double-stranded DNA termini was conducted according to Gubler's method using a cDNA kit (Amersham, U.K.)

### (5) Preparation of cDNA library

After protecting possible EcoRI cleavage sites in the double-stranded cDNA prepared in (4) above by EcoRI methylase, providing both ends with EcoRI linker, it was integrated into lambda-gt11 DNA at the EcoRI site and assembled with phage protein to make recombinant phage. Series of this reaction was processed using the lambda-gt11 cloning kit (Amersham, U.K.). Library size of cDNA primed by oligo dT₁₂ and that of cDNA primed by randam-hexanucleotide were 1.0 x 10⁶ PFU and 4.3 x 10⁶ PFU respectively.

### (6) Screening of cDNA library

cDNA libraries prepared under (5) were searched for a non-A, non-B hepatitis related cDNA clone by immunoscreening. After infecting E.coli Y1090 with the above recombinant phage, and dispensing it on LB-Agar plates for incubation at 43°C for 3 hours, a filter impregnated with 1PTG was placed on it and incubated at 37°C for 3 hours. The filter was then removed and washed with the buffer and primary antibody was added to it. A mixture of plasma of a chimpanzee infected with non-a, non-B hepatitis, a human plasma known to have caused non-A, non-B hepatitis by needle stick accident, and a plasma of chronic non-A, non-B hepatitis patient were used as the primary antibody, and incubated at 4°C overnight. After washing with the buffer and addition of the secondary antibody (peroxidase labeled antihuman IgG), it was incubated at room temperature for 30 minutes. It was then washed with the buffer and added with the mixture of DAB (3,3′-Diaminobenzidine tetrahydrochloride; Sigma, US), Ni, Co, H₂O₂ for color development. If there were fractions of non-A, non-B related genes in the cDNA libraries, and if they fused with the open reading frame (ORF) of β-galactosidase of lambda-gut11 phage DNA in-frame, there should be fusion protein expressed by E.coli infected with the phage and, upon its recognition by non-A, non-B related antibody, it would bind to the antibody to give a positive signal. Inventors' experiment confirmed that point.

Among several clones giving positive signals in the screening test, there was a clone named GOR47-1 which is the basis of this invention.

### (7) Purification of fusion protein made by non-A, non-B hepatitis related cDNA clone GOR47-1 and lambda-gt11 β-galactosidase

E. .coli Y1089 was lysogenically infected with the non-A, non-B hepatitis related gene (GOR47-1) obtained under (6) above to make lysogen. (Lysogen was prepared according to the method described in "Constructing and Screening cDNA Libraries in lambda-gt11", Thanh V. Huyuh et al., DNA Cloning Vol. 1, a practical approach, ed. by D.M. Clover, 949-78, IRL Press, Oxford, 1985.) After culturing and inducing expression of protein by IPTG (isoprogyl-β-D-thiogalactoside), and destruction of the lysogen by means of freezing-thawing and sonication, lysate was obtained and subjected to an affinity chromatography column (Sepharose 4B Immobilized IgG Fraction Rabbit anti-β-galactosidase; Cappel, U.S.) to obtain the fusion protein.

### (8) Detection of non-A, non-β hepatitis related antibody by the fusion protein of GOR47-1 and β-galactosidase

The fusion protein obtained under (7) above was electrophoresed by SDS-PAGE and its pattern was transferred onto nitrocellulose membrane by the Western blotting technique. Sample plasma was applied onto this membrane and after incubation anti-human immunoglobulin labeled with peroxidase was added for detection of the antibody through color reaction.

The nitrocellulose membrane with the electrophoresed pattern transferred onto it was rinsed for 10 minutes, dried naturally, shredded into strips 3.5 mm wide, and those strips were immersed in TBS (50mM Tris-Cl buffer, pH 7.4, 150 mM NaCl) containing 2% skim milk at room temperature for 1 hour or at 4°C overnight (blocking), then washed with TBST (TBS containing 0.05% Tween 20). After immersion at room temperature for 1 hour or at 4°C overnight in the primary antibody diluted 30 times with 3% BSA (primary antigen-antibody reaction), those strips were washed with TBST, immersed at room temperature for 30 minutes in biotinylated anti-human IgG or anti-human IgM (Vectastain ABC kit; Vector Laboratories Inc., US) diluted with TBS (secondary antigen-antibody reaction), washed with TBST, then immersed in peroxidase labeled mixture of biotin and avidin (Vectastain ABC kit; Vector Laboratories Inc., US) at room temperature for 30 minutes (biotin-avidin reaction), then washed with TBST followed finally by addition of coloring reagent to examine the presence of reactive substances. The coloring reagent was prepared by adding 25 mg of DAB (3,3′-Diaminobenzidine tetrahydrochloride; Sigma, US) to 50 ml of 50 µM NAPO₄ pH7.4 and, after dissolution of DAB, 1 ml of 5% CoCl₃-6H₂O, 5% (NH₄)₂Ni(SO₄ )₂·6H₂O, then, 50 µl of 30% H₂O₂. When antibody was positive, a band of about 120 K Dalton was stained dark blue.

### (9) Determination of the nucleotide sequence of non-A, non-B hepatitis related cDNA clone (GOR47-1)

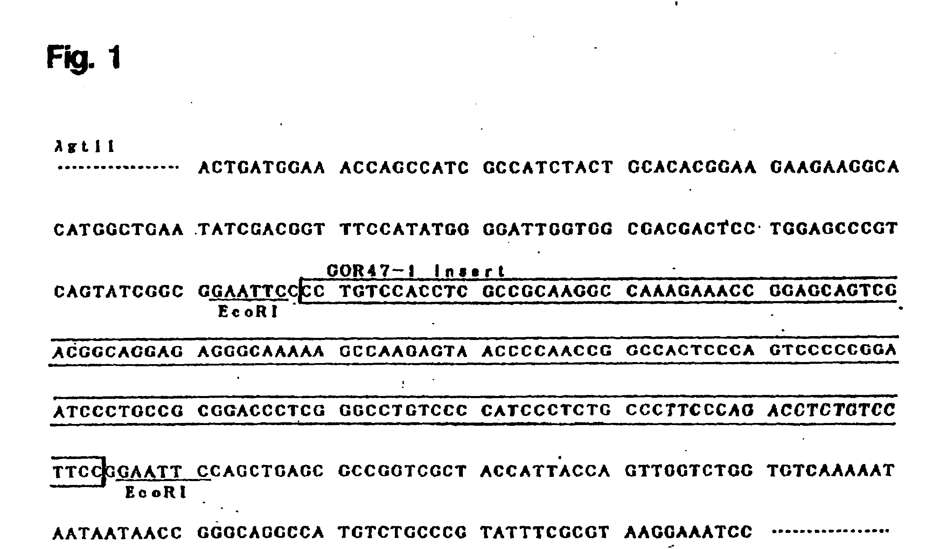

The nucleotide sequence of GOR47-1 obtained under (6) above was determined by Sanger's method by purifying it, cleaving its DNA by EcoRI to take out cDNA, and subcloning it to EcoRI site of Phagescript (trade name of Stratagene, US). By means of primer derived from DNA phage, the nucleotide sequence at the insertion site of GOR47-1 was simultaneously determined in the same method. As a result, it was confirmed that GOR47-1 DNA was inserted into lambda-gt11 DNA by the sequence and direction shown in Fig. 1. Fig. 1 shows the nucleotide sequence of GOR47-1 with the junctional sequence of lambda-gt11 DNA. Sequence within the box is for GOR47-1 and those on the left and right sides of the box are for the lambda phage.

### (10) Determination of the primary structure of the Protein coded for by non-A, non-B hepatitis related cDNA clone (GOR47-1)

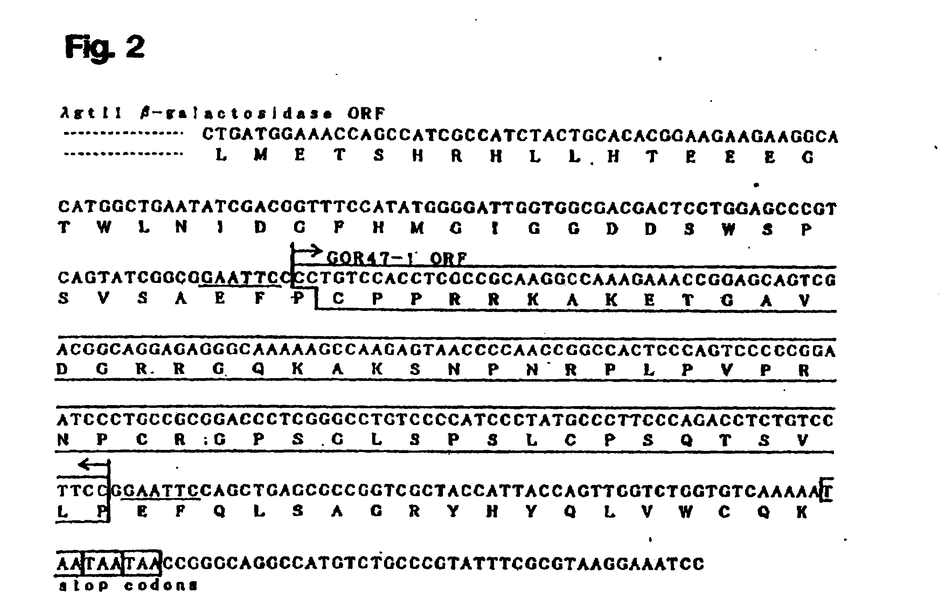

From the nucleotide sequence determined under (9) above, ORF of GOR47-1 linked with ORF of β-galactosidase of lambda-gt11 in frame was determined as per Fig. 2. Lambda-gt11 phage DNA used for preparation of cDNA libraries from which GOR47-1 was derived is a so called expression vector and expresses fusion protein of β-galactosidase and cDNA derived protein by assembling CDNA in operon of lacZ gene of lambda-gt11. Out of 6 possible reading frames, only one frame can fuse in frame with ORF of β-galaccosidase of lambda-gt11 as shown in Fig. 2. There is no stop codon in this ORF encoding 55 amino acids. Under this invention, β-galactosidase can be replaced by such expression proteins as alkaline phosphatase and superoxide dismutase.

### (11) Search for cDNA clone overlapping in sequence with non-A. non-B hepatitis related cDNA clone (GOR47-1)

From GOR47-1 phage obtained under (6) above, GOR47-1 DNA and GOR47-1 DNAC were cut out as insert DNA, and from recombinant phagescript obtained under (9) above, GOR47-1 RNA and GOR47-1 RNAC were prepared by T₈ and T₇ promoters. They were all labeled with radioisotope. When they were used as probes to screen the cDNA libraries prepared under (5) above, cDNA clone (GORS22 DNA), while overlapping with GOR47-1, had a longer sequence. The base sequence of GORS22 shown below was determined in the same way an described under (9) above (underlined sequence shows the common sequence with GOR47-1 DNA):

C G A G A G C T C A C C C T G T C C A C
C T C G C C G C A A G G C C A A A G A A
A C C G G A G C A G T C G A C G G C A G
G A G A G G G C A A A A A G C C A A G A
G T A A C C C C A A C C G G C C A C T C
C C A G T C C C C C G G A A T C C C T G
C C G C G G A C C C T C G G G C C T G T
C C C C A T C C C T C T G C C C T T C C
G A G A C C T C T G T C C T T C C A C T
A A T C G C C T C C C G C A G C A C C G
A G C C G C C A C T C C C A G T C C C C
C G A G T C C C T G C C G C G C C C C C
T C G C G C C T G T C C A C A T C C C T
C T G C C C A T C C G A G A C C T C T G
T C C T T A C A C C A C T A G C C A C C
C C A C G T G G A C T T C C A T G G C
C T C T G A G T A C A A G G C C A G C C
C C C C G G C C C A C C A G C T T T C T
G A A T G T G T G C T T A.

When compared with GOR47-1 PROTEIN under (10) above, the amino acid sequence of the non-A, non-B hepatitis related protein coded for by GORS22 DNA was as follows (underlined sequence shows common sequence with GOR47-1 PROTEIN):

E S S P C P P R R K A K E T G A V D G R

R G Q K A K S N P N R P L P V P R N P C
R G P S G L S P S L C P S Q T S V L P L
I A S R S T E P P L P V P R V P A A P P
R A C P H P S A H P R P L S L H H.

### (12) Determination of non-A, non-B hepatitis related epitope by synthetic peptide based on the amino acid sequence coded for by non-A, non-B hepatitis related cCNA clone (GORS22 DNA), and assay of non-A, non-B hepatitis related antibody by synthetic peptide

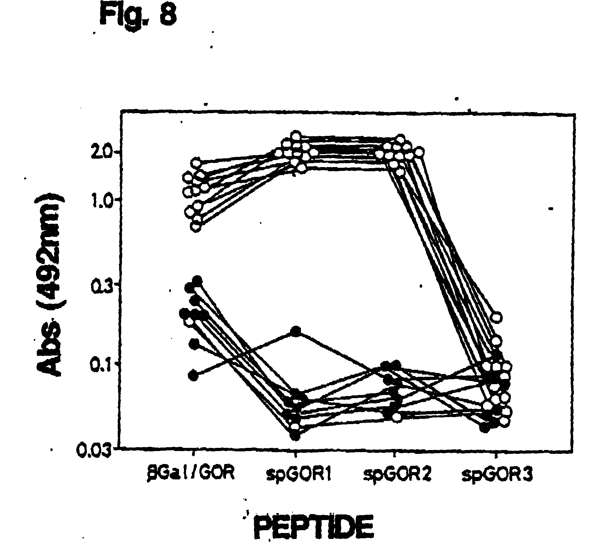

Various peptides were synthesized based on the sequence of the ORF of GORS22 and examined for their reactivity with non-A, non-B hepatitis related antibody to determine the location of the non-A, non-B hepatitis epitope. Polystyrene microplate coated with synthetic peptide prepared by Merrifield's solid phase method served as solid phase, sample sera served as solid primary antibody, and peroxidase labeled anti-human IgG or IgM antibodies served as secondary antibody for color reaction. As a result, it has been found that the oligopeptide spGOR1 and the oligopeptide spGOR2 have the common antigenic epitope. It has also been found that the non-A, non-B hepatitis related epitope is located on the oligopeptide spGOR-epi, overlapping sequence of spGOR1 and spGOR2 (Fig 8). Samples turned out positive (Abs 492 0.4) to β-galactosidase/GOR, spGOR1 and spGOR2 have been confirmed NANB-GOR antibody positive by Western blot analysis. An example of Enzyme Immuno Assay (EIA) for detection of non-A, non-B hepatitis specific antibody using the spGOR2 will be discussed below.

### (13) Preparation of antibody specific to non-A, non-B hepatitis related antigen

Monoclonal and polyclonal antibodies were obtained by immunizing mice, guinea pigs, goats, horses, etc. with synthetic peptide bearing the epitope of non-A, non-B hepatitis antigen determined under (12) above.

### (14) Preparation of antigen assay by specific antibody to the non-A, non-B hepatitis related antigen

Antibodies prepared under (13) above were labeled with FITC (fluorescein isothiocyanate) to make a probe for staining sample sections, such as liver tissue of patients infected with non-A, non-B hepatitis, to locate or examine locality of antigen specific to non-A, non-B hepatitis in tissue. An assay system was also developed by labelling specific antibodies with peroxidase or biotin to detect non-A, non-B hepatitis related antigen in patient's sera or plasma. This assay employs the "sandwich method" in which microplates or beads are coated with specific antibodies, and samples and labeled antibodies are applied in sequence for reaction.

### (15) Detection of non-A, non-B hepatitis related gene by Polymerase Chain Reaction (PCR)

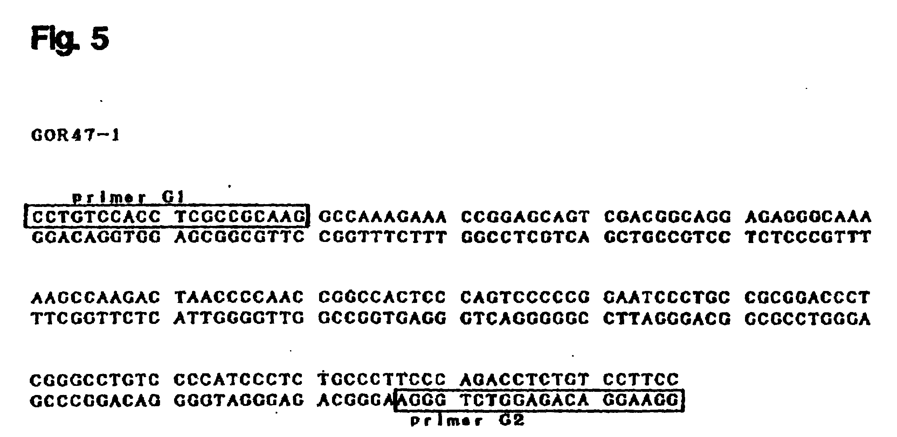

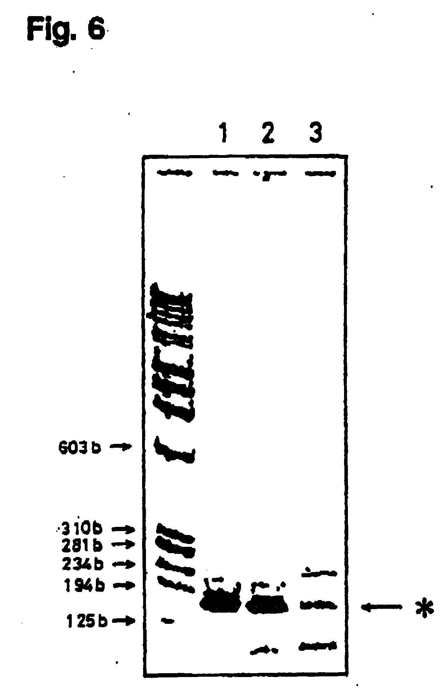

According to the method described under (3) above, viral fractions were obtained by centrifugation of sample sera or plasma and subjected to PCR after treatment with SDS/Proteinase K cocktail and extraction of nucleic acid with phenol. Primer used for PCR was oligonucleotide G1 and G2, each consisting of 20 nucleotides as shown in Fig. 5 and located at both ends of non-A, non-B hepatitis related cDNA GOR47-1. G1 corresponds to 20 bases of 5' terminus of GOR47-1 DNA and is closest to 5' side of the sense strand, while G2 corresponds to 20 bases of 5' terminus of GOR47-1 DNAC and is closest to 5' side of anti-sense strand. After addition of grimer G2 (antisense) to the above mentioned nucleic acid fractions as the primary reaction in PCR, 4 nucleotides and transcriptase were added for reaction and synthesization of primary cDNA. Primer G1 was then added and the standard PCR reaction was followed for 36 cycles using Taq polymerase and the automatic temperature control unit (Cetus Corp., U.S.). The product (DNA) thus produced was separated by agarose gel electrophoresis and the presence of an expected length (same 166 bases as GOR47-1) of DNA fragment (identified by * in Fig. 6) was confirmed. Chimpanzee (CH19) plasma from which non-A, non-B hepatitis related cDNA clone GOR47-1 was derived contained PCR product of 166 bases (Fig. 6, Lane 1). A similar band was separated from the plasma of chimpanzee (CH413) having known infectious unit of 10⁷ CIU/ml (chimpanzee infectious units/ml) (Fig. 6, Lane 2). Human plasma (T.S) known to have 10⁶ CIU/ml also showed the similar length of band but its amount was about 1/10 of that of the aforementioned chimpanzee having 10⁷ CIU/ml. Patterns on the extreme left of the figure are those of molecular markers.

### (16) Difference between HCV genome by Chiron corporation and non-A, non-B hepatitis related cDNAs GOR47-1 and GORS22

There is neither more than 50% homology noted between the nucleotide sequence of the previously mentioned HCV made public by Chiron Corporation and the nucleotide sequences of GOR47-1 and GORS22, nor more than 50% homology noted between the amino acid sequence of HCV made public by Chiron Corporation and the amino acid sequences coded for by GOR47-1 and GORs22.

### Examples

(A) An example of the assay for detection of non-A, non-B hepatitis related antibody using GOR47-1 PROTEIN and fusion protein as antigen is described below. In the experiment, fusion protein of GRO47-1 PROTEIN and lambda-gt11 β-galactosidase was used.

### (1) Detectability of non-A, non-B hepatitis related antibody (antibody that can be detected by antigen under this invention is hereafter called NANB-GOR antibody) in chronic hepatitis patients with chronic liver diseases

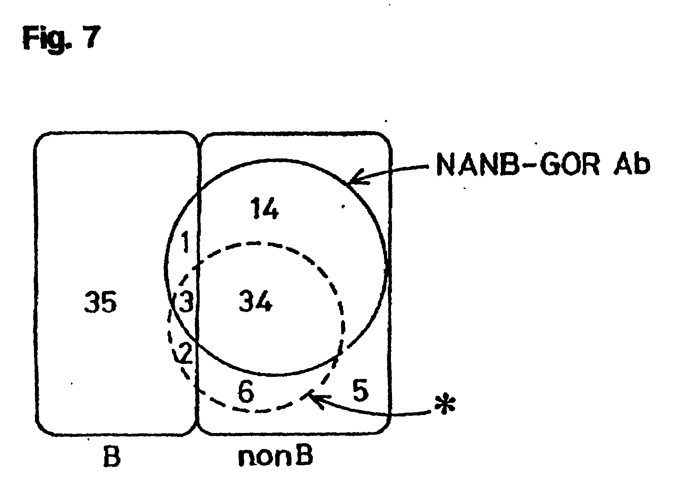

Sera of 100 patients with chronic liver diseases (40 cases of hepatitis B and 60 cases of non-B hepatitis) were assayed by the Western blot analysis described under (8) above for NANB-GRO antibody. Results are shown in Tables 1 and 2, and Fig. 7. As shown in Tables 1 and 2, among 100 cases, NANB-GOR antibody was positive for 52 cases in total and its rate was much higher for the sera of non-B hepatitis cases (80%) than for the sera of hepatitis B cases (10%).
Especially in chronic non-B hepatitis cases, NANB-GOR antibody was positive for 27 cases out of 30 cases (90%). When NANB-GOR antibody was compared with Chiron's HCV antibody (Ortho HCV Ab ELISA test; Ortho Diagnostic Systems, Tokyo, Japan), the former showed lower positive rate for the sera of hepatitis B cases (10%, 13%) and conversely higher positive rate for sera of non-A, non-B hepatitis cases (80%, 68%) than the latter. Furthermore, as shown in Fig. 7, there were 14 non-A, non-B hepatitis cases which NANB-GOR antibody detected but HCV antibody failed to detect, while there were only 6 converse cases.

### (2) NANB-GOR antibody in sera from donors having abnormal liver function

Frequency of NANB-GOR antibody and HCV antibody for sera of randomly selected 41 donors (ALT over 80, hepatitis B negative) were, as shown in Tables 3, 20% and 17% respectively. Sera were tested in the method described in (1) immediately above. In five cases, both NANB-GOR antibody and HCV antibody were positive. ALT (alanine aminotransferase) level in serum is one of the markers which indicate liver function and is high (abnormal) when host is infected with viral hepatitis, but it is also high when liver is malfunctioning due to some other causes such as alcohol, drugs, obesity, etc. The group consisting of 41 donors was consciously healthy but was unconsciously high in ALT level and differed from the groups in (1) above in that the cause of the high ALT level was not checked in this group while that of the groups in (1) was checked (chronic hepatitis, liver cirrhosis, or hepatoma). The difference in frequency of NANB-GOR antibody between this group and those in (1) may be attributed to this fact.

### (3) Seroconversion of NANB-GOR antibody in acute non-A, non-B hepatitis patient

It is already been demonstrated that the frequency of NANB-GOR antibody was high for patients with non-B type chronic liver diseases (acute hepatitis, liver cirrhosis, hepatoma). It has also been found that it seroconverts at a very early stage of acute hepatitis. Table 4 contains data from a nurse who had been accidentally exposed to a needle stick contaminated with blood from a non-A, non-B hepatitis patient resulting in an acute hepatitis. As the table shows, there was no NANB-GOR antibody before infection and it was detected only after infection. At the initial stage of infection, NANB-GOR antibody of the IgM class was also positive. Emergence of HCV antibody, on the other hand, was very late. At the 8th week after infection, NANB-GOR antibody was already positive, while Chiron's HCV antibody was negative (Ortho HCV Ab ELISA test; Ortho Diagnostic Systems, Tokyo, Japan) until the 17th week after infection. This privies that, at least for this case, serological diagnostic of non-A, non-B hepatitis infection at an early stage is possible only with NANB-GOR antibody. Expression of IgM class NANB-GOR antibody in the acute phase presents another important significance. It is not easy to discern between an acute exacerbation of chronic hepatitis and acute hepatitis. They can, however, be distinguished by testing for NANB-GOR antibody IgM class and accurate diagnostic becomes possible. (B) Next example shows Enzyme Immuno Assay (EIA) for detection of the non-A, non-B hepatitis related antibody using an antigen the synthetic oligopeptide spGOR2 containing non-A, non-B hepatitis related epitode.

spGRO2 synthesized by the method described by Merrifield (1969) was dissolved in Tris-CHl buffer (10mM, pH7.5) to make 5 µg/ml concentration. Then, 50 µl of it was dispensed in each well of a Costar vinyl plate (Toyobo, Osaka, Japan) and incubated overnight at room temperature (coating of microplate wells with peptide for preparation of solid phase). After washing wells (0.1% Tween 20, 150mM NaCl), 100 µl of blocking buffer (0.1% Tween 20, 150mM NaCl, 30% Bovine Fetal Serum) was dispensed to each well and incubated overnight at 4°C. After washing wells, 50 µl of sample serum or plasma diluted 30 fold in readiness with the aforementioned blocking buffer was dispensed to each well and was constantly agitated for 30 minutes at room temperature. Wells were washed again and 50 µl of peroxidase labeled anti-human IgG or IgM mouse monoclonal antibody solution was dispensed in each well, and agitated for 30 minutes at room temperature. Wells were washed and peroxidase substrate solution for color reaction for absorbance measurement at 492nm was dispensed in each well. Results of experiments thus obtained are shown below.

### (1) NANB-GOR antibody detected in chronic hepatitis patients:

### Comparison of EIA using synthetic peptide spGOR2 and Western blot analysis using protein fused with β-galactosidase

100 sera from patients with chronic tyge-B or type-NANB liver diseases were tested by EIA for NANB-GOR antibody and most of them turned out to be positive by both EIA and Western blot analysis, but 11 samples were positive by EIA but negative or weakly positive by Western blot analysis, suggesting that the former detects NANB-GOR antibody at higher sensitivity than the latter (Table 5).

### (2) Detection of NANB-GOR antibody in normal subjects or patients with various chronic diseases: EIA using spGOR2

As Fig. 9 shows, NANB-GOR antibody frequency detected by EIA Was as high as 76%, 56% and 56% for non-A, non-B related chronic hepatitis, liver cirrhosis and hepatoma respectively, while it was as low as 2%, 7% and 0% for hepatitis B related chronic hepatitis, liver cirrhosis and hepatoma respectively. It was 0% and 0% for lupoid hepatitis and primary biliary cirrhosis which were supposed to be closely associated with autoimmune mechanism, or was only 2 (1%) out of 200 for normal subjects. This proved a close relation of NANB-GOR antibody with non-A, non-B hepatitis.

### (3) Detection of NANB-GOR antibody in an acute non-A, non-B hepatitis patient: EIA using spGOR2

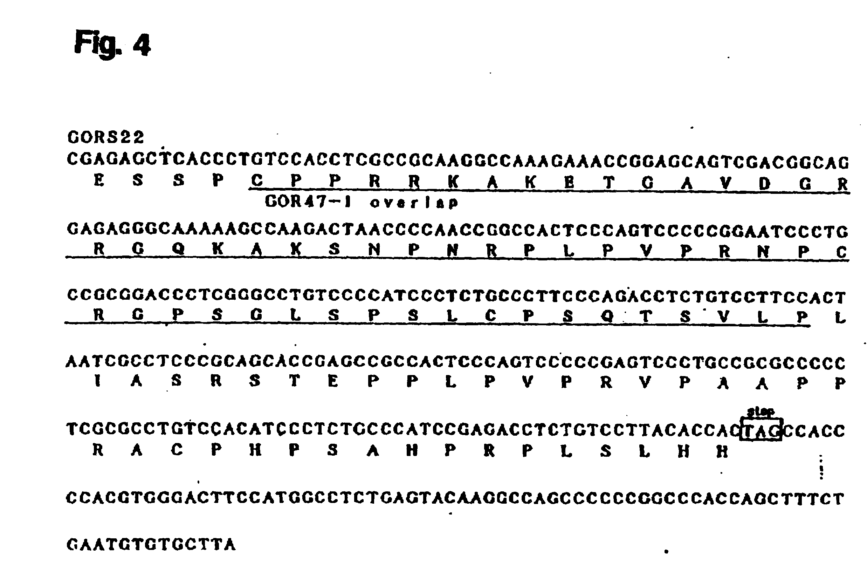

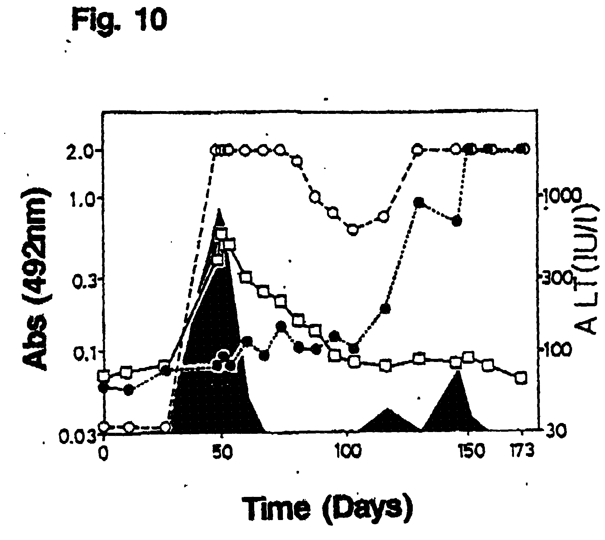

Of the patient with non-A, non-B hepatitis shown in Fig. 4, IgM and IgG classes NANB-GOR antibodies were determined. Fig. 10 shows the results.

In this particular case, both IgM and IgG class of NANB-GOR antibodies were detected on the 49th day after the needle stick accident while HCV antibody was detected (Ortho HCV Ab ELISA test; Ortho Diagnostic Systems, Japan) as late as on the 131st day after the accident. This has proven that NANB-GOA antibody is effective in early detection of infection.

The antigens and antibodies of the invention provide diagnostic reagents capable of quicker and wider range of diagnosis of non-A, non-β hepatitis than any other conventional reagents, and can be used at blood centers, blood derivatives manufacturers, transfusion departments of hospitals and many other places for elimination of blood carrying non-A, non-B hepatitis agent from transfusion blood and blood derivatives. Screening tests by the diagnostic reagents using the antigen and antibodies under this invention will provide optimum means for prevention of post transfusion hepatitis which has long been a grave concern.

The nucleic acids and protein under this invention are manufactured not only as important reagents for serological and virological study of non-A, non-B hepatitis causative agent and for pathological study of the disease caused by it, but also as indispensable materials for manufacture of the aforementioned antigens and antibodies.

EP-A-0 377 303 indicates that the materials of the present invention may be utilized in detection kits and as vaccines.

It is well known in the art that one or more amino acids in an amino acid sequence can be replaced by equivalent other amino acids, as demonstrated by U.S. Patent No. 4,737,487 in order to produce an analog of the amino acid sequence.

Further variations and modifications of the invention will become apparent to those skilled in the art from the foregoing and are intended to be encompassed by the claims appended hereto.

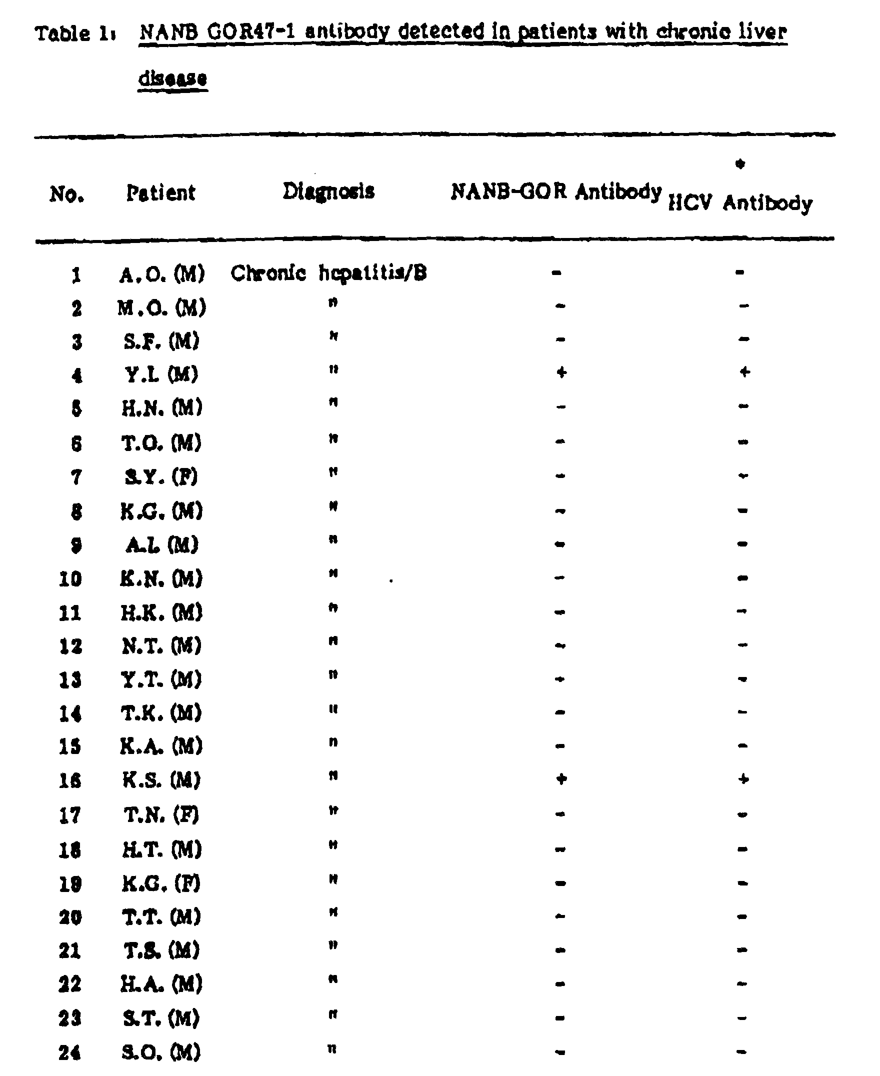

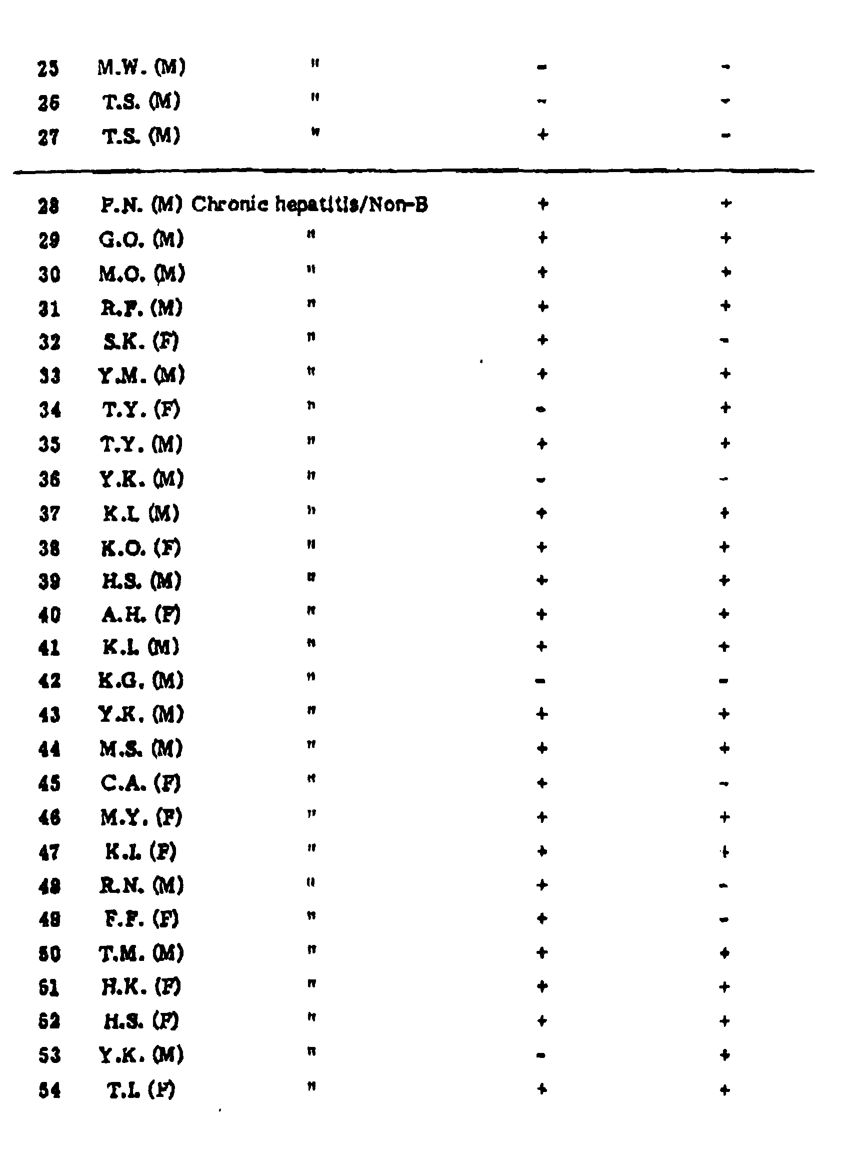

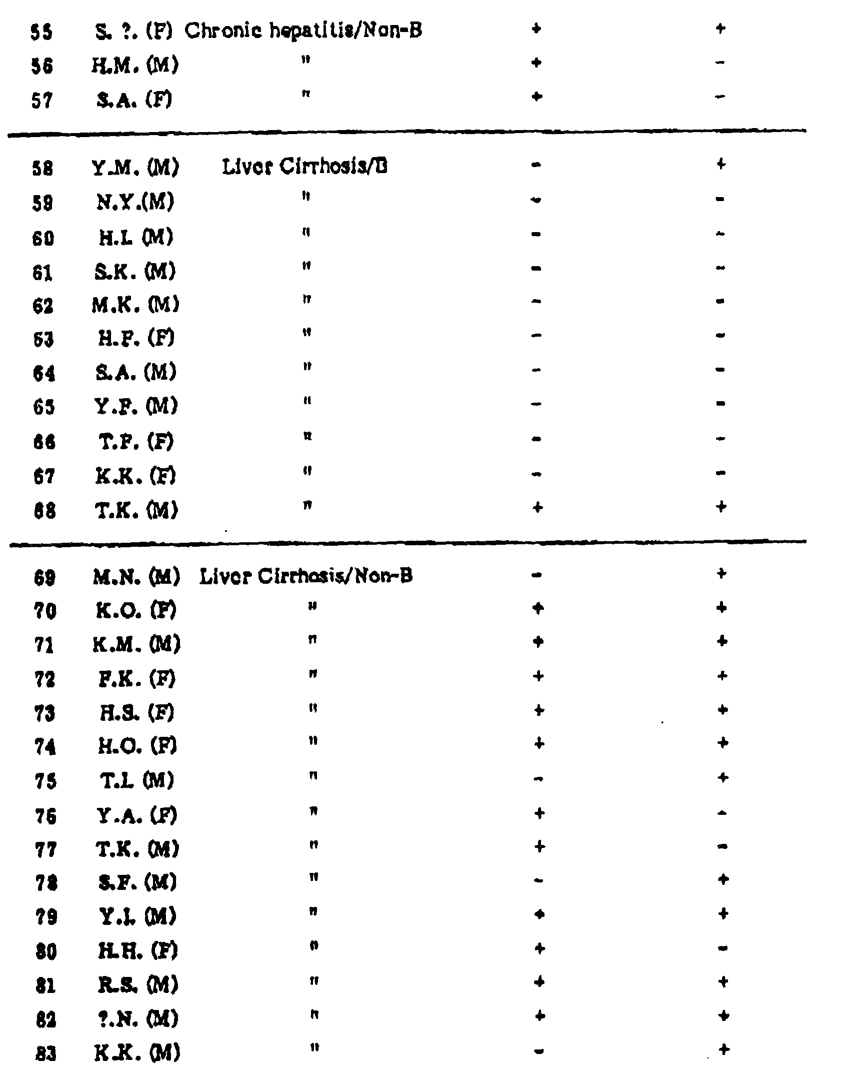

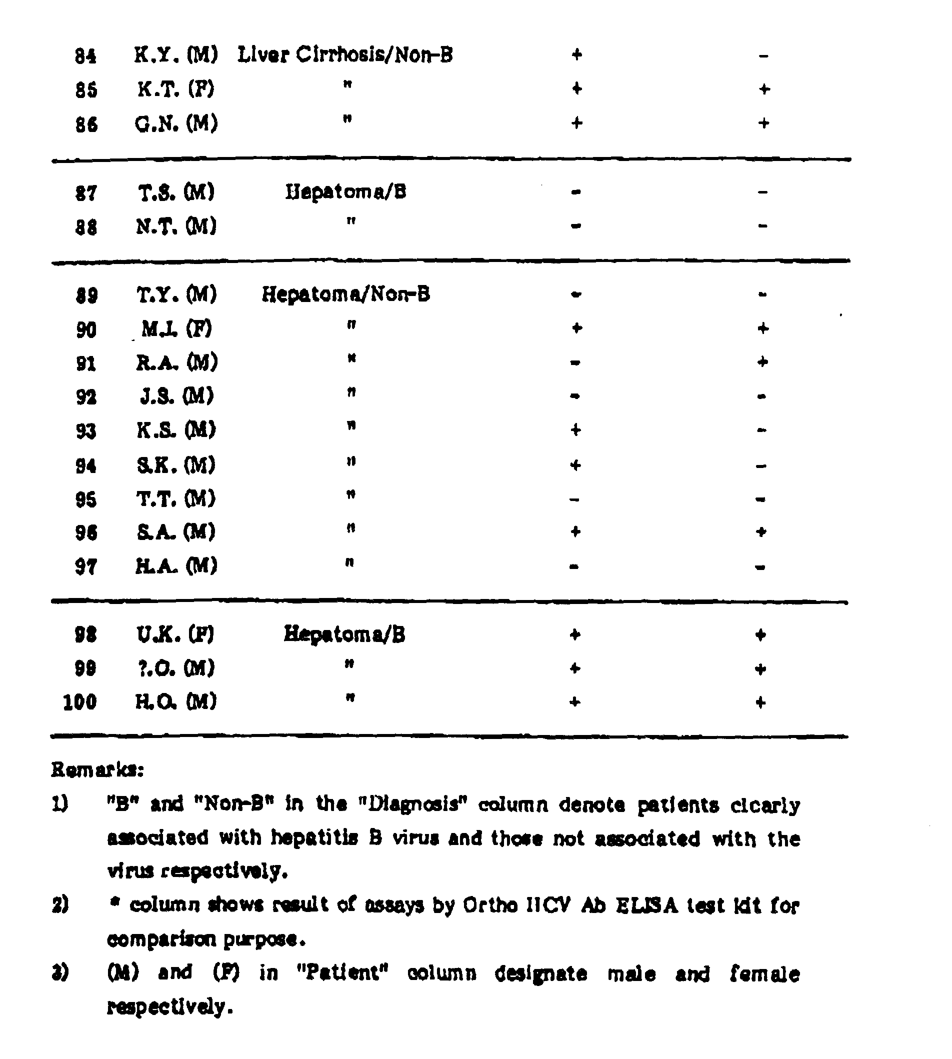

**Table 2:**

| Frequency of NANB GOR47-1 antibody in patients with liver diseases | | | |
|---|---|---|---|
| Disease Group | Number of Patient | Frequency of NANB-GOR Antibody Number (Rate) | * HCV Antibody |
| Chronic hepatitis/B | 27 | 3(11%) | 2(7%) |
| Chronic hepatitis/Non-B | 30 | 27(90%) | 21(70%) |
| Liver cirrhosis/B | 11 | 1(9%) | 3(27%) |
| Liver cirrhosis/Non-B | 18 | 14(78%) | 14(78%) |
| Hepatoma/B | 2 | 0(0%) | 0(0%) |
| Hepatoma/Non-B | 12 | 7(58%) | 6(50%) |
| B Total | 40 | 4(10%) | 5(13%) |
| Non-B Total | 60 | 48(80%) | 41(68%) |
| Grand Total | 100 | 52(52%) | 46(46%) |

| | | | |
|---|---|---|---|
| Remarks: * column shows for comparison number (rate) of samples tested positive for HCV in Table 1. | | | |

**Table 3:**

| NANB-GOR47-1 antibody detected in donors with malfunctioning liver. | | | | | |
|---|---|---|---|---|---|
| Donor No. | NANB-GOR Antibody | * HCV Antibody | Donor No. | NANB-GOR Antibody | * HCV Antibody |
| 1 | - | - | 22 | - | - |
| 2 | - | - | 23 | + | + |
| 3 | - | - | 24 | + | - |
| 4 | - | - | 25 | - | + |
| 5 | - | - | 26 | - | - |
| 6 | - | - | 27 | - | - |
| 7 | - | - | 28 | - | - |
| 8 | - | - | 29 | - | - |
| 9 | - | - | 30 | + | + |
| 10 | - | - | 31 | - | - |
| 11 | - | - | 32 | + | + |
| 12 | - | - | 33 | - | - |
| 13 | - | - | 34 | - | - |
| 14 | - | - | 35 | - | - |
| 15 | - | + | 36 | + | - |
| 16 | - | - | 37 | - | - |
| 17 | - | - | 38 | + | + |
| 18 | - | - | 39 | - | - |
| 19 | - | - | 40 | - | - |
| 20 | - | - | 41 | N.T. | - |
| 21 | + | + | 42 | + | - |
| Remarks: 1) For comparison purpose, * column shows the result of samples tested for Chiron's HCV antibody. 2) All samples were tested negative for hepatitis B virus antigen. An demonstrated in Table 2, 90% of Non-B chronic hepatitis samples were tested positive for NANB-GOR antibody, while only 20% of those "consciously healthy" popuration were positive for the antibody since such liver malfunction derives not only from the virus but from obesity and alcohol. 3) "N.T." means "not tested". | | | | | |

**Table 4:**

| Seroconversion of NANB-GOR47-1 in an acute non-A, non-B hepatitis patient | | | | |
|---|---|---|---|---|
| Time (Week) | ALT | NANB-GOR Antibody | | * (HCV Antibody) |
| | | IgM | IgG | |
| 0 | 13 | - | - | - |
| 4 | 32 | N.T. | N.T. | - |
| 7 | 889 | N.T. | N.T. | - |
| 8 | 381 | + | + | - |
| 11 | 5 | + | ± | - |
| 14 | 20 | N.T. | N.T. | - |
| 17 | 41 | + | - | - |
| 19 | 15 | N.T. | N.T. | + |
| 21 | 77 | N.T. | N.T. | + |
| 22 | 38 | + | - | + |
| 27 | 18 | + | - | + |
| 250 | N.T. | ± | N.T. | N.T. |
| Remarks: 1) Above table shows a case of a nurse infected with hepatitis by needle prick accident. Time indicates number of week(s) elapsed after the accident. the patient, the source or the blood collected, also tested positive for NANB-GOR47-1 antibody. 2) Result of the tests for Chiron's HCV are shown in * column for comparison. 3) N.T. means "not tested". | | | | |

**Table 5:**

| Comparison of EIA using spGOR2 antibody and Western blot analysis using fusion protein | | | | | | | | | | | |
|---|---|---|---|---|---|---|---|---|---|---|---|
| No. | EIA | West. | No. | EIA | West. | No. | EIA | West. | No. | EIA | West. |
| 1 | 28 | - | 26 | >2000 | + | 51 | 1603 | + | 76 | 53 | - |
| 2 | 57 | - | 27 | 71 | - | 52 | 53 | - | 77 | >2000 | + |
| 3 | 107 | - | 28 | 63 | - | 53 | 55 | - | 78 | >2000 | + |
| 4 | >2000 | + | 29 | 1557 | + | 54 | 1554 | + | 79 | >2000 | + |
| 5 | 474 | - | 30 | 37 | - | 55 | 52 | ± | 80 | >2000 | + |
| 6 | 50 | - | 31 | 34 | - | 56 | 1512 | + | 81 | 386 | ± |
| 7 | 134 | - | 32 | 87 | - | 57 | 910 | - | 82 | >2000 | + |
| 8 | >2000 | + | 33 | >2000 | + | 58 | >2000 | + | 83 | 1554 | + |
| 9 | 1662 | - | 34 | 102 | - | 59 | 35 | - | 84 | 896 | ± |
| 10 | 51 | - | 35 | 63 | - | 60 | 110 | ± | 85 | >2000 | + |
| 11 | 807 | ± | 36 | 61 | - | 61 | 29 | - | 86 | 49 | - |
| 12 | 62 | - | 37 | 32 | - | 62 | 1941 | + | 87 | 75 | - |
| 13 | 101 | - | 38 | 1039 | + | 63 | 55 | - | 88 | 1630 | + |
| 14 | 99 | - | 39 | 30 | - | 64 | 789 | + | 89 | 33 | - |
| 15 | 1886 | + | 40 | 1731 | + | 65 | >2000 | + | 90 | 1283 | + |
| 16 | >2000 | + | 41 | 49 | - | 66 | 1945 | + | 91 | >2000 | + |
| 17 | 77 | - | 42 | 265 | ± | 67 | 53 | - | 92 | 35 | - |
| 18 | >2000 | + | 43 | >2000 | + | 68 | 200 | - | 93 | 64 | - |
| 19 | 397 | ± | 44 | 51 | - | 69 | 39 | - | 94 | >2000 | + |
| 20 | 80 | - | 45 | 98 | - | 70 | 119 | - | 95 | 101 | ± |
| 21 | 63 | - | 46 | 34 | - | 71 | 167 | - | 96 | >2000 | + |
| 22 | >2000 | + | 47 | >2000 | + | 72 | 1582 | + | 97 | 69 | ± |
| 23 | >2000 | + | 48 | >2000 | + | 73 | 469 | - | 98 | 729 | ± |
| 24 | >2000 | + | 49 | 87 | - | 74 | >2000 | + | 99 | >2000 | + |
| 25 | 68 | - | 50 | >2000 | + | 75 | 607 | + | 100 | 807 | ± |
| Remarks: "West." indicates Western blot analysis. | | | | | | | | | | | |

## Claims

1. Recombinant ribonucleic acid GOR47-1 RNA comprising the following nucleotide sequence:

C C U G U C C A C C U C G C G C G A A G

G C C A A A G A A A C C G G A G C A G U

C G A C G G C A G G A G A G G G C A A A

A A G C C A A G A G U A A C C C C A A C

C G G C C A C U C C C A G U C C C C C G

C G G G C C U G U C C C C A U C C C U C

U G C C C U U C C C A G A C C U C U G U

C C U U C C.

2. Recombinant ribonucleic acid GOR47-1 RNAC comprising the following nucleotide sequence:

G G A A G G A C A G A G G U C U G G G A
A G G G C A G A G G A U G G G A C A
G G C C C G A G G G U C C G C G G C A G
G G A U U C C G G G G A C U G G G A G
U G G C C G G U U G G G U U A C U C U
U G G C U U U U U G C C C U C U C C U G
C C G U C G A C U G C U C C G G U U U C
U U U G G C C U U G C G G C G A G G U G
G A C A G G.

3. Recombinant deoxyribonucleic acid GOR47-I DNA comprising the following nucleotide sequence:

C C T G T C C A C C T C G C C G C A A G
G C C A A A G A A A C C G G A G C A G T
C G A C G G C A G G A G A G G G C A A A
A A G C C A A G A G T A A C C C C A A C
C G G C C A C T C C C A G T C C C C C G
G A A T C C C T G C C G C G G A C C C T
C G G G C C T G T C C C C A T C C C T C
T G C C C T T C C G A G A C C T C T G T
C C T T C C.

4. Recombinant deoxyribonucleic acid GOR47-1 DNAC comprising the following nucleotide sequence:

G G A A G G A C A G A G G T C T G G G A
A G G G C A G A G G G A T G G G G A C A
G G C C C G A G G G T C C G C G G C A G
G G A T T C C G G G G A C T G G G A G
T G G C C G G T T G G G G T T A C T C T
T G G C T T T T G C C C T C T C C T G
C C G T C G A C T G C T C C G G T T T G
T T T G G C C T T G C G G C G A G G T G
G A C A G G.

5. A protein GOR47-1 PROTEIN comprising the following amino acid sequence:

C P P R R K A K E T G A V D G R R G Q K
A K S N P N R P L P V P R N P C R G P S
G L S P S L C P S Q T S V L P or analogs
thereof.

6. An oligopeptide spGOR1 comprising the following amino acid sequence:

C P P R R K A K E T G A V D G R R G Q K
A K S N P N R P L or analogs thereof.

7. An oligopeptide spGOR2 comprising the following amino acid sequence:

G R R G Q K A K S N P N R P L P V P R N
P C R G P S G or analogs thereof.

8. An oligopeptide spGOR-epi comprising the following amino acid sequence:

G R R G Q K A K S N P N R P L or analogs
thereof.

9. A non-A, non-B hepatitis related nucleic acid detection reagent comprising the nucleic acid according to any of claims 1 to 4 as primer or hybridization probe.

10. A non-A, non-B hepatitis antibody detection reagent comprising the amino acid sequence according to any of claims 5 to 8 as antigen.

11. A non-A, non-B hepatitis related antibody detection reagent comprising a fusion protein of the amino acid sequence according to any of claims 5 to 8 and a conventional protein for its expression.

12. A non-A, non-B hepatitis specific monoclonal or polyclonal antibody reactive with an antigen comprising the amino acid sequence according to any of claims 5 to 8.

13. A non-A, non-B hepatitis related antigen detection reagent comprising a specific antibody according to claim 12.

14. An immunoassay for detecting antibodies directed against non-A, non-B hepatitis antigens, comprising the amino acid sequence according to claim 5 or claim 7.

15. An immunoassay for detecting non-A, non-B hepatitis antigens, comprising a non-A, non-B hepatitis specific monoclonal or polyclonal antibody according to claim 12.

16. A non-A, non-B hepatitis diagnostic test kit comprising the nucleic acid according to claim 1 as probe or hybridization probe.

17. A non-A, non-B hepatitis diagnostic test kit comprising the non-A, non-B hepatitis specific monoclonal or polyclonal antibody according to claim 12.

18. A non-A, non-B hepatitis diagnostic test kit comprising the amino acid sequence according to claim 5.

19. A non-A, non-B hepatitis diagnostic test kit comprising a fusion protein of the amino acid sequence according to claim 5 and a conventional protein for its expression.

20. A method for detecting non-A, non-B hepatitis nucleic acids in a sample, comprising:
(a) reacting nucleic acids of the sample with a probe for a non-A, non-B hepatitis polynucleotide under conditions which allow the formation of a polynucleotide duplex between the probe and the non-A, non-B hepatitis nucleic acid from the sample; and
(b) detecting a polynucleotide duplex which contains the probe; wherein said probe has the following nucleotide sequence:

C C U G U C C A C C U C G C G C G A A G
G C C A A A G A A A C C G G A G C A G U
C G A C G G C A G G A G A G G G C A A A
A A G C C A A G A G U A A C C C C A A C
C G G C C A C U C C C A G U C C C C C G
C G G G C C U G U C C C C A U C C C U C
U G C C C U U C C C A G A C C U C U G U
C C U U C C.

## Patentansprüche

1. Rekombinante Ribonukleinsäure GOR47-1 RNA mit folgender Nukleotidsequenz:

C C U G U C C A C C U C G C G C G A A G
G C C A A A G A A A C C G G A G C A G U
C G A C G G C A G G A G A G G G C A A A
A A G C C A A G A G U A A C C C C A A C
C G G C C A C U C C C A G U C C C C C G
C G G G C C U G U C C C C A U C C C U C
U G C C C U U C C C A G A C C U C U G U
C C U U C C.

2. Rekombinante Ribonukleinsäure GOR47-1 RNAC mit folgender Nukleotidsequenz:

G G A A G G A C A G A G G U C U G G G A
A G G G C A G A G G G A U G G G G A C A
G G C C C G A G G G U C C G C G G C A G
G G A U U C C G G G G A C U G G G A G
U G G C C G G U U G G G G U U A C U C U
U G G C U U U U U G C C C U C U C C U G
C C G U C G A C U G C U C C G G U U U C
U U U G G C C U U G C G G C G A G G U G
G A C A G G.

3. Rekombinante Desoxyribonukleinsäure GOR47-I DNA mit folgender Nukleotidsequenz:

C C T G T C C A C C T C G C C G C A A G
G C C A A A G A A A C C G G A G C A G T
C G A C G G C A G G A G A G G G C A A A
A A G C C A A G A G T A A C C C C A A C
C G G C C A C T C C C A G T C C C C C G
G A A T C C C T G C C G C G G A C C C T
C G G G C C T G T C C C C A T C C C T C
T G C C C T T C C G A G A C C T C T G T
C C T T C C.

4. Rekombinante Desoxyribonukleinsäure GOR47-1 DNAC mit folgender Nukleotidsequenz;

G G A A G G A C A G A G G T C T G G G A
A G G G C A G A G G A T G G G A C A
G G C C C G A G G T C C G C G G C A G
G G A T T C C G G G G A C T G G G A G
T G G C C G G T T G G G T T A C T C T
T G G C T T T T T G C C C T C T C C T G
C C G T C G A C T G C T C C G G T T T G
T T T G G C C T T G C G G C G A G G T G
G A C A G G.

5. Protein GOR47-1 PROTEIN mit folgender Aminosäuresequenz: 
C P P R R K A K E T G A V D G R R G Q K
A K S N P N R P L P V P R N P C R G P S
G L S P S L C P S Q T S V L P
 oder Analoge davon.

6. Oligopeptid spGOR1 mit folgender Aminosäuresequenz: 
C P P R R K A K E T G A V D G R R G Q K
A K S N P N R P L
 oder Analoge davon.

7. Oligopeptid spGOR2 mit folgender Aminosäuresequenz: 
G R R G Q K A K S N P N R P L P V P R N
P C R G P S G
 oder Analoge davon.

8. Oligopeptid spGOR-epi mit folgender Aminosäuresequenz: G R R G Q K A K S N P N R P L oder Analoge davon.

9. Non-A, Non-B Hepatitis betreffendes Nukleinsäure-Nachweisreagenz, enthaltend die Nukleinsäure gemäß einem der Ansprüche 1 bis 4 als Primer oder Hybridisierungssonde.

10. Non-A, Non-B Hepatitis-Antikörper-Nachweisreagenz, enthaltend die Aminosäuresequenz gemäß einem der Ansprüche 5 bis 8 als Antigen.

11. Non-A, Non-B Hepatitis betreffendes Antikörper-Nachweisreagenz, enthaltend ein Fusionsprotein aus der Aminosäuresequenz gemäß einem der Ansprüche 5 bis 8 und einem konventionellen Protein zu seiner Expression.

12. Non-A, Non-B Hepatitis-spezifischer monoklonaler oder polyklonaler Antikörper, welcher mit einem Antigen enthaltend die Aminosäuresequenz gemäß einem der Ansprüche 5 bis 8 reagiert.

13. Non-A, Non-B Hepatitis betreffendes Antigen-Nachweisreagenz enthaltend einen spezifischen Antikörper gemäß Anspruch 12.

14. Immunoessay zum Nachweis von Antikorpern gegen Non-A, Non-B Hepatitisantigene, enthaltend die Aminosäuresequenz gemäß anspruch 5 oder Anspruch 7.

15. Immunoessay zum Nachweis von Non-A, Non-B Hepatitisantigenen, enthaltend einen Non-A, Non-B Hepatitis-spezifischen monoklonalen oder polyklonalen Antikörper gemäß Anspruch 12.

16. Non-A, Non-B Hepatitis diagnostischer Testkit, enthaltend die Nukleinsäure gemäß Anspruch 1 als Sonde oder Hybridisierungssonde.

17. Non-A, Non-B Hepatitis diagnostischer Testkit, enthaltend den Non-A, Non-B Hepatitis-spezifischen monoklonalen oder polyklonalen Antikörper gemäß Anspruch 12.

18. Non-A, Non-B Hepatitis diagnostischer Testkit, enthaltend die Aminosäuresequenz gemäß Anspruch 5.

19. Non-A, Non-B Hepatitis diagnostischer Testkit, enthaltend ein Fusionsprotein aus der Aminosäuresequenz gemäß Anspruch 5 und einem konventionellen Protein zu seiner Expression.

20. Methode zum Nachweis von Non-A, Non-B Hepatitis-Nukleinsäuren in einer Probe mit den Stufen:
(a) Reagieren von Nukleinsäuren der Probe mit einer Sonde für ein Non-A, Non-B Hepatitis-Polynukleotid unter Bedingungen, die die Bildung eines Polynukleotiddoppelstranges zwischen der Sonde und der Non-A, Non-B Hepatitis-Nukleinsäure aus der Probe erlauben, und
(b) Nachweis eines Polynukleotiddoppelstranges, welcher die Sonde enthält, wobei die Sonde folgende Nukleotidsequenz besitzt:

C C U G U C C A C C U C G C G C G A A G.
G C C A A A G A A A C C G G A G C A G U
C G A C G G C A G G A G A G G G C A A A
A A G C C A A G A G U A A C C C C A A C
C G G C C A C U C C C A G U C C C C C G
C G G G C C U G U C C C C A U C C C U C
U G C C C U U C C C A G A C C U C U G U
C C U U C C.

## Revendications

1. Acide ribonucléique recombinant ARN GOR47-1 comprenant la séquence nucléotidique suivante :

C C U G U C C A C C U C G C G C G A A G
G C C A A A G A A A C C G G A G C A G U
C G A C G G C A G G A G A G G G C A A A
A A G C C A A G A G U A A C C C C A A C
C G G C C A C U C C C A G U C C C C C G
C G G G C C U G U C C C C A U C C C U C
U G C C C U U C C C A G A C C U C U G U
C C U U C C.

2. Acide ribonucléique recombinant ARNC GOR47-1 comprenant la séquence nucléotidique suivante :

G G A A G G A C A G A G G U C U G G G A
A G G G C A G A G G G A U G G G G A C A
G G C C C G A G G U C C G C G G C A G
G G A U U C C G G G G G A C U G G G A G
U G G C C G G U U G G G U U A C U C U
U G G C U U U U U G C C C U C U C C U G
C C G U C G A C U G C U C C G G U U U C
U U U G G C C U U G C G G C G A G G U G
G A C A G G.

3. Acide désoxyribonucléique recombinant ADN GOR47-I comprenant la séquence nucléotidique suivante :

C C T G T C C A C C T C G C C G C A A G
G C C A A A G A A A C C G G A G C A G T
C G A C G G C A G G A G A G G G C A A A
A A G C C A A G A G T A A C C C C A A C
C G G C C A C T C C C A G T C C C C C G
G A A T C C C T G C C G C G G A C C C T
C G G G C C T G T C C C C A T C C C T C
T G C C C T T C C G A G A C C T C T G T
C C T T C C.

4. Acide désoxyribonucléique recombinant ADNC GOR47-1 comprenant la séquence nucléotidique suivante :

G G A A G G A C A G A G G T C T G G G A
A G G G C A G A G G G A T G G G G A C A
G G C C C G A G G G T C C G C G G C A G
G G A T T C C G G G G G A C T G G G A G
T G G C C G G T T G G G G T T A C T C T
T G G C T T T T T G C C C T C T C C T G
C C G T C G A C T G C T C C G G T T T G
T T T G G C C T T G C G G C G A G G T G
G A C A G G.

5. Protéine GOR47-1 comprenant la séquence d'acides aminés suivante : 
C P P R R K A K E T G A V D G R R G Q K
A K S N P N R P L P V P R N P C R G P S
G L S P S L C P S Q T S V L P
 ou analogues de celle-ci.

6. Oligopeptide spGOR1 comprenant la séquence d'acides aminés suivante : 
C P P R R K A K E T G A V D G R R G Q K
A K S N P N R P P L
 ou analogues de celui-ci.

7. Oligopeptide spGOR2 comprenant la séquence d'acides aminés suivante : 
GRRGQKAKSNPNRPLPVPRN
PCRRGPSG
 ou analogues de celui-ci.

8. Oligopeptide épi-spGOR comprenant la séquence d'acides aminés suivante : GRRGQKAKSNPNRPL ou analogues de celui-ci.

9. Réactif de détection d'acide nucléique associé à l'hépatite non A, et à l'hépatite non B, comprenant l'acide nucléique selon l'une quelconque des revendications 1 à 4, en tant qu'amorce ou sonde d'hybridation.

10. Réactif de détection d'anticorps d'hépatite non A et d'hépatite non B, comprenant la séquence d'acides aminés selon l'une quelconque des revendications 5 à 8, en tant qu'antigène.

11. Réactif de détection d'anticorps associé à l'hépatite non A et à l'hépatite non B, comprenant une protéine de fusion de la séquence d'acides aminés selon l'une quelconque des revendications 5 à 8, et une protéine conventionnelle pour son expression.

12. Anticorps monoclonal ou polyclonal spécifique de l'hépatite non A et de l'hépatite non B, réactif avec un antigène comprenant la séquence d'acides aminés selon l'une quelconque des revendications 5 à 8.

13. Réactif de détection d'antigène associé à l'hépatite non A et à l'hépatite non B, comprenant un anticorps spécifique selon la revendication 12.

14. Dosage radio-immunologique pour la détection d'anticorps dirigés contre les antigènes d'hépatite non A et non B, comprenant la séquence d'acides aminés selon la revendication 5, ou la revendication 7.

15. Dosage radio-immunologique pour la détection d'antigènes comprenant un anticorps monoclonal ou polyclonal spécifique de l'hépatite non A et de l'hépatite non B, selon la revendication 12.

16. Kit de test diagnostic d'hépatite non A et d'hépatite non B, comprenant l'acide nucléique selon la revendication 1, en tant qu'amorce ou sonde d'hybridation.

17. Kit de test diagnostic d'hépatite non A et d'hépatite non B, comprenant un anticorps monoclonal ou polyclonal spécifique de l'hépatite non A et de l'hépatite non B, selon la revendication 12.

18. Kit de test diagnostic d'hépatite non A et d'hépatite non B, comprenant la séquence d'acides aminés selon la revendication 5.

19. Kit de test diagnostic d'hépatite non A et d'hépatite non B, comprenant une protéine de fusion de la séquence d'acides aminés selon la revendication 5, et une protéine conventionnelle pour son expression.

20. Méthode de détection d'acides nucléiques d'hépatite non A et d'hépatite non B, dans un échantillon, et comprenant :
(a) la réaction des acides nucléiques de l'échantillon avec une sonde pour un polynucléotide d'hépatite non A et d'hépatite non B, dans des conditions permettant la formation d'un duplex de polynucléotide entre la sonde, et l'acide nucléique d'hépatite non A et d'hépatite non B provenant de l'échantillon et
(b) la détection d'un duplex de polynucléotide qui contient la sonde dans lequel ladite sonde a la séquence nucléotidique suivante :
